# NEW EUROPEAN PATENT SPECIFICATION

(11) **EP 1 897 532 B2**
(45) Date of publication and mention of the opposition decision: **05.11.2014**
(45) Mention of the grant of the patent: 09.03.2011
(21) Application number: 06018938.8
(22) Date of filing: 11.09.2006
(51) Int. Cl.: A61K 8/42, A61Q 5/10

(54) **Hair colouring composition**
Haarfärbemittel
Teinture pour cheveux

(43) Date of publication of application: 12.03.2008
(73) Proprietor: Kao Germany GmbH, 64297 Darmstadt (DE)
(72) Inventor: Uellner, Martin, 64295 Darmstadt (DE); Kufner, Frank, 64297 Darmstadt (DE); Blumenschein, Katrin, 64753 Brombachtal-Hembach (DE)

(56) References cited:
- EP-A- 1 627 666
- EP-A2- 1 430 874
- WO-A-00/07550
- WO-A2-03/013450
- WO-A2-03/041671
- JP-A- H0 320 209
- US-A1- 2004 146 478
- US-A1- 2006 064 824

## Description

Present invention relates to a process for oxidative colouring hair.

Bleaching and oxidative colouring hair is a common practice in hair dressing area. Especially the female users of hair colouring products tend to change their hair colour considerably often and time to time present various wishes which gives or can give certain difficulties. Such difficulties are especially observed when hair is considerably damaged by previous or in the same process right before oxidative colouring hair performed chemical treatments especially bleaching. One of them is difficulties in achieving so called warm shades in an oxidative colouring hair. With the term "warm shade" it is meant that hair colour thus obtained comprises a red, reddish touch. In other words when a hair colour is expressed with its "L", "a" and "b" values as measured by a laboratory colour measuring device wherein "L" being the intensity of the colour, "a" being the intensity of red colour and "b" being the intensity of the yellow colour, both "a" and "b" values are having a positive value independent from the colour intensity.

In practice when colouring hair especially multiple processed hair and more specifically at least twice bleached hair with an oxidative dyeing compositions into a warm shade the resulting colour is often not satisfactorily warm enough. In other words, the colour obtained does not have red or reddish touch as expressed with a positive "a" value. In order to overcome such results, obtained colours either have to be corrected by means of application of another colouring composition, or application of a pre-colouration composition as well comprising hair colorants either oxidative or direct, or mixing colouring composition with an another colouring composition prior to application. All of the mentioned options are currently being the usual practice at hair dressing salons. Even the latter one has further drawbacks as it is solely based on the hair dressers existing colour analysis.

Furthermore, it has also been observed the colours obtained lack of homogeneity as roots and tips having different colour intensity. Moreover, stability of colours so obtained especially against shampooing is varying largely from root to tip of the hair.

In addition, another problem is hair condition after colouration service. Especially the chemically multiple treated hair and in particular bleached or more than once bleached hair can usually not be combed well, does not feel smooth and soft upon touching so that additional products must be applied right after colouration which is often time consuming and increases the price of a service.

JP-A-2004107247 patent application discloses hair colouring and conditioning compositions comprising a fatty alcohol, a nonionic surfactant, an amido amine compound and a basic dye.

WO-A-03/013450 discloses cosmetic compositions based on oxidative dye precursors and coupling substances comprising at least a branched fatty alcohol and a nonionic emulsifier.

The aim of the present invention is to overcome above mentioned problems and provide an oxidative hair colouring process for colouring hair especially chemically multiple processed and bleached or more than once bleached hair, especially into warm colour shades.

The present inventors have surprisingly found out that a process for oxidative colouring hair based on oxidative dye precursors and coupling substances comprising at least one branched fatty alcohol, at least one non-ionic emulsifier and at least one compound of the following formula 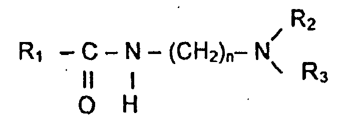 wherein R1 is an saturated or unsaturated alkyl chain having 11 to 23 C atoms, R₂ and R₃ are the same of different an alkyl chain having 1 to 4 C atoms and n is a number between 1 and 4 and/or its salts, colours hair intensive, homogeneous and especially into warm shades without any need for using pre-treatments or shade mixings, and hair coloured with such composition is easier to comb through and feels soft and smooth upon touching.

At least one branched fatty alcohol is contained in compositions at a concentration of 1 to 20%, preferably 2 to 15% and more preferably 2.5 to 10% by weight calculated to total composition prior to mixing with an oxidizing agent comprising composition.

In principal any branched fatty alcohol having a total carbon number of 14 to 30, preferably 14 to 24 are suitable for the compositions. Some examples are octyldecanol, octyldodecanol, isocetyl and isostearyl alcohols. The most preferred one is octyldodecanol.

An amido amine compound according to the formula given above is contained in the composition at a concentration of 0.1 to 10%, preferably 0.5 to 7.5%, more preferably 1 to 7.5% and most preferably 2 to 5% by weight calculated to total composition prior to mixing with an oxidizing agent comprising composition.

Any amidoamine compound having an alkyl chain of 12 to 24 C including the carbonyl C of the amide bond is suitable for the compositions. Examples are stearamidopropyldimethylamine, Palmitamidopropyldiethylamine, Palmitamidopropyldimethylamine, Behenamidopropyldimethylamine and /or their salts.

At least one non-ionic emulsifier is included into the compositions at a concentration of 0.5 to 15%, preferably 1 to 10%, more preferably 1 to 7,5% by weight calculated to total composition prior to mixing with an oxidizing agent comprising composition.

The preferred non-ionic emulsifiers are ethoxylated fatty alcohols with an alkyl chain of 12 to 24 C atoms and with number of ethoxyl groups of 2 to 50, preferably 10 to 30. Examples are ceteth-20, seteareth-30, palmeth-20, steareth -20, beheneth-20 etc. These compounds are named according to the fatty alcohol they are originating and number of ethoxyl groups is given at the end. These compounds are well known emulsifiers and found in any cosmetic ingredient book.

Further suited nonionic surfactants are, especially in mixture with fatty alcohol ethoxylates, for example, long-chain fatty acid mono- and dialkanolamides, such as coco fatty acid mono- or diethanolamide and myristic fatty acid mono or diethanolamide, stearic acid mono or diethanolamide.

Further nonionic surfactants suited again especially in admixture with fatty alcohol ethoxylates mentioned above are alkyl polyglucosides of the general formula

R₆-O-(R₃O)ₙ-Zₓ,

wherein R₆ is an alkyl group with 8 to 18 carbon atoms, R₃ is an ethylene or propylene group, Z is a saccharide group with 5 to 6 carbon atoms, n is a number from 0 to 10 and x is a number between 1 and 5.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ - alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx^{®}", "Aromox^{®}" or "Genaminox^{®}".

The composition comprises at least one oxidative dye precursor. Some examples are p-phenlynediamine, p-methylaminophenol and substituted p-phenylenediamines such as 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, pyrazole and the derivatives thereof such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene or the water-soluble salts thereof.

Further suitable aminopyridines are 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(fi-hydroxyethyl amino)-6-methoxy-pyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof.

Further, Indole and indoline derivatives can as well be contained in the colouring composition of the present invention. Examples to those are: 6-aminoindol, 6-hydroxyindole, 1-ethyl-6-hydroxyindole, 1-methyl-4-hydroxyindol, 1-methyl-6-hydroxyindole, 2-methyl-6-hydroxyindole, 5-hydroxyindol, 4-hydroxyindol, 5,6-dihydroxyindole, 6-aminoindoline, 6-hydroxyindoline, 1-ethyl-6-hydroxyindoline, 1-methyl-4-hydroxyindoline, 1-methyl-6-hydroxyindoline, 2-methyl-6-hydroxyindoline, 5-hydroxyindoline, 4-hydroxyindoline, 5,6-dihydroxyindoline and their respective salts.

The composition comprises at least one coupling substance. Suitable ones are resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxypyridine, 2-dimethyl-amino-5-aminopyridine, 2,6-diaminopyridine, 1,3-diaminobenzene, 1-amino-3-(2'-hy-droxyethylamino)benzene, 1-amino-3-[bis(2'-hydroxyethyl) amino]benzene, α-naphthol, 4,6-dichlororesorcinol, 1,3-diamino-toluene, 1-hydroxy naphthalene, 4-hydroxy-1 ,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1.2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof. 5-amino-2-methylphenol, 2-methyl-5-hydroxyethylaminophenol, 2,4,-diamnophenoxyehanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol, or the water-soluble salts thereof.

Total concentration of oxidative dye precursors and coupling substances are in the range of 0.01 to 10% by weight, preferably 0.05 to 7.5% by weight and more preferably 0.1 to 5% by weight, calculated to total composition prior to mixing with an oxidizing agent. Weight ratio of one or more oxidative dye precursors to one or more coupler substance is in the range of 1:8 to 8:1, preferably 1:5 to 5:1 and more preferably 1:2 to 2:1.

Compositions may also comprise direct dyes. Direct dyes are found to be useful for adjusting any colour tone within the meaning of the present invention.

Direct dyes can be cationic, anionic and/or nitro dyes. Some examples to cationic ones are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green, Basic Orange 31, 1, Basic Red 2, Basic Red 12 Basic Red 22, Basic Red 76 Basic Red 51, Basic Violet 1, Basic Violet 2, Basic Violet 3, Basic Violet 10, Basic Violet 14 and Basic Yellow 57 Basic Yellow 87.

Examples to anionic ones are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No. 15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Concentration of direct dyes is in total is in the range of 0.01 to 2.5% by weight calculated to total composition prior to mixing with oxidizing agent.

pH of the composition is in the range of 5 to 12, preferably 6 to 11 and more preferably 6.8 to 10, in particular 6.8 to 7 after mixing with an oxidizing agent.

Compositions is mixed prior to application onto hair with a composition comprising at least one oxidizing agent. The preferred oxidizing agent is hydrogen peroxide at a concentration of 2 to 12% by weight. Other peroxides such as urea peroxide, and melanin peroxide is also possible to use.

Subject of the present invention is process for colouring hair especially damaged hair wherein a composition comprising at least one oxidative dye precursor and at least one coupling substance and further comprising at least one branched fatty alcohol, at least one amido amine compound of the above formula and at least one non-ionic emulsifier is mixed with an oxidizing composition comprising at least one oxidizing agent and applied onto hair and processed for 10 to 45 min at a temperature of 20 to 45°C and rinsed off from hair.

Colouring composition can comprise additionally in the base formulation fatty acids with 0 to 3 ethylenic bonds and with fatty acyl chain length of 12 to 22 C atom. Concentration of the fatty acids can be in the range of 0.1 to 10%, preferably 0.1 to 7.5% and most preferably 0.2 to 5% by weight calculated to the total composition. Fatty acid examples, without limiting the choice, suitable for colouring compositions are myristic acid, palmitic acid, behenic acid, steraic acid, oleic acid, linoleic acid. The most preferred fatty acid is oleic acid.

Colouring compositions are emulsions.

In addition to the branched fatty alcohol colouring composition comprises an additional fatty alcohol or mixture of fatty alcohols with the chain length of 14 to 22 C atoms. Examples to suitable fatty alcohols, without limiting the choice, are myristyl alcohol, cetyl alcohol, stearyl alcohol, behenyl alcohol and cetostearyl alcohol. The most preferred is cetostearyl alcohol well known with its trade name Lanette O or as Lanette N in mixture with sodium cetearyl sulfate from Cognis.'Total fatty alcohol content should be in the range of 1 to 20% by weight, calculated to total composition priot to mixing with an oxidizing agent.

Colouring compositions comprises surfactants selected from anionic, amphoteric (or zwiterionic) and/or cationic surfactants as emulsifier or solubilizer. Cationic surfactants are as well used as hair conditioners in the colouring composition.

Anionic surfactants suitable within the scope of the invention are in principal known from the cleansing compositions and may be present in an amount from 0.1 to about 10%by weight, calculated to the total composition prior to mixing with an oxidizing agent. Compatibility of anionic surfactant in the composition should be taken into account when choosing the type and the concentration.

These are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₅- (C₂H₄O)ₙ- O - CH₂COOX,

wherein R₅ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 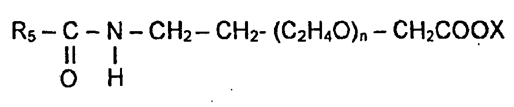 wherein R₅ and X have the above meanings, and n is in particular a number from 1 to 10, preferably 2.5 to 5.

Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants in a mixture.

An overview of the anionic surfactants suitable for the present invention can furthermore be found in the monography of K. Schrader, "Grundlagen und Rezepturen der Kosmetika", 2nd Ed.(1989, Hüthig Buchverlag), pp. 595-600 and pp. 683 to 691.

As further surfactant component, the colouring compositions can also contain amphoteric or zwitterionic surfactants, for example in an amount from about 0.5 % to about 5 %, preferably from about 1 % to about 2.5 % by weight, calculated to the total composition.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

Colouring composition can comprise cationic surfactants as emulsifier, solubilizer and/or conditioning ingredients according to the formula, 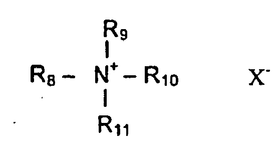 where R₈ is a saturated or unsaturated, branched or non-branched alkyl chain with 8-22 C atoms or

R₁₂ CO NH (CH₂)ₙ

where R₁₂ is saturated or unsaturated, branched or non-branched alkyl chain with 7-21 C atoms and n has typical value of 1 - 4 or

R₁₃ CO O (CH₂)ₙ

where R₁₃ is saturated or unsaturated, branched or non-branched alkyl chain with 7-21 C atoms and n has typical value of 1 - 4, and
R₉ is H or unsaturated or saturated, branched or non-branched alkyl chain with 1 - 22 C atoms or

R₁₂ CO NH (CH₂)ₙ

or

R₁₃ CO O (CH₂)ₙ

where R₁₂, R₁₃ and n are same as above.
R₁₀ and R₁₁ are H or lower alkyl chain with 1 to 4 Carbon atoms, and X is typically chloride, bromide, methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Form the above mentioned surfactants in addition to the non-ionic surfactant, the most preferred surfactant type is cationic surfactant.

Colouring composition can also contain cationic polymers as conditioning agents. Those are cationic cellulose type polymers know as Polymer JR type from Amerchol such as Polyquaternium 10 or cationic guar gum known with trade name Jaguar from Rh6ne-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46. Among those the most preferred one is the Polyquaternium 11 as well known with its trade name Gafquat from ISP and as Luviquat PQ from BASF.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

Typical concentration range for any of the cationic conditioners mentioned above can be 0.01 - 5% by weight, preferably 0.03 - 2.5% by weight and more preferably 0.05 - 1.5% by weight.

Hair dyeing composition preferably comprise an organopolysiloxane wherein at least one silicium atom is linked to an alkylene group having a hetero-atom, in particular a nitrogen atom, with a poly-(N-acyl alkyleneimine) units of the formula 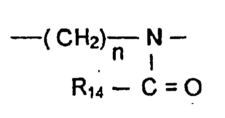 wherein n is a number from 1 to 5 and R₁₄ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 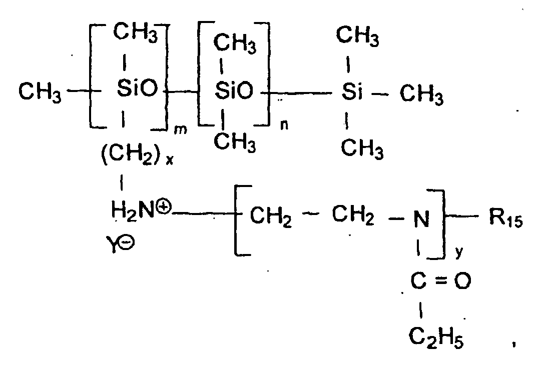 wherein m and n each are numbers from 20 to 10,000, in particular 50 to 7,000, especially 100 to 5,000, x is a number between 1 and 5, preferably 3, and y is a number from 5 to 30, R₁₅ is a C₁-C₁₂-alkyl or aryl group, in particular a methyl, ethyl or benzyl group, and Y⁻ is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Colouring compositions can contain organic solvents as penetration enhancers and also as a solubilzers. Examples of such organic solvents are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. Typically the concentration of those solvents can be in the range from 0.5% to 10%, preferably 0.5 - 5% by weight calculated to the total composition.

The hair dyeing compositions may comprise thickening agents. These are, for example, the various cellulose derivatives such as hydroxyalkyl celluloses, e.g. hydroxyethyl cellulose, hydroxypropyl cellulose, hydroxypropyl methyl cellulose, natural polysaccharides such as xanthan gum; guar gum and the alkoxylation products thereof in amounts from 0.1 - 5 %, preferably 0.1 - 3% and most preferably 0.1 - 2% by weight calculated to the total composition and depending on the desired consistency thereof.

Optionally, the colouring composition can comprise further hair conditioning agents such as silicone oils either volatile or non-volatile, natural and synthetic oils. Among silicone oils those can be added to the colouring composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Additional non-ionic conditioning agents may be polyols such as glycerin, glycol and derivatives, polyethyleneglycoles known with trade names Carbowax PEG from Union Carbide and Polyox WSR range from Amerchol, polyglycerin and polyethyleneglycol mono or di fatty acid esters

Colouring composition may further comprise ceramide type compound. Such compounds are especially beneficial for their repair effects which is disclose in number of patents and/or application from the applicant.

Compositions of the present invention can further comprise ingredients customarily found in such compositions such as alkalizing agents preservatives antioxidants, fragrances, reducing agents and chelating agents.

The following examples are to illustrate the present invention.

### Example 1

| | % by weight |
|---|---|
| Octyldodecanol | 10.0 |
| Propylene glycol | 4.0 |
| Ceteareth-30 | 5.0 |
| Stearamidopropyldimethylamine | 5.0 |
| Polysilicone-9 | 0.1 |
| Panthenol | 0.5 |
| Sodium sulfite | 0.5 |
| Ascorbic acid | 0.2 |
| Tetrasodium EDTA | 0.2 |
| Fragrance, preservative | q.s |
| Ethanolamine | 0.3 |
| p-toluenediamine sul fate | 0.43 |
| 2,5,6-triamino-4-pyrimidinol sulphate | 0.01 |
| Resorcinol | 0.01 |
| m-aminophenol | 0.07 |
| 2-amino-3-hydroxypyridine | 0.03 |
| 4-amino-m-cresol | 0.02 |
| Water | q.s. to 100 |

The above composition was mixed with a composition comprising hydrogen peroxide at a concentration of 2% by weight at a weight ratio of 1:1 which was resulted in a pH value of 6.9 and was applied onto a twice bleached hair. After processing of 30 min at 30°C the hair was rinsed with tap water and shampooed once with a commercial shampoo and dried with a hair drier. A shiny warm homogeneous colour was obtained. The L, a and b values measured at the root and tip parts as identified on the tress used were not significantly differing from each other showing clearly that hair is homogeneously coloured. In addition the hair feel soft and smooth upon touching and was easily combed through.

### Example 2

| | % by weight |
|---|---|
| Octyldodecanol | 10.0 |
| Ceterayl alcohol | 2.0 |
| Propylene glycol | 4.0 |
| Ceteareth-30 | 5.0 |
| Stearamidopropyldimethylamine | 4.0 |
| Cetyltrimonium chloride | 1.0 |
| Polysilicone-9 | 0.1 |
| Panthenol | 0.5 |
| Sodium sulfite | 0.5 |
| Ascorbic acid | 0.2 |
| Tetrasodium EDTA | 0.2 |
| Fragrance, preservative | q.s |
| Ethanolamine | 0.3 |
| p-toluenediamine sul fate | 0.43 |
| 2,5,6-triamino-4-pyrimidinol sulphate | 0.01 |
| Resorcinol | 0.01 |
| m-aminophenol | 0.07 |
| 2-amino-3-hydroxypyridine | 0.03 |
| 4-amino-m-cresol | 0.02 |
| Water | q.s. to 100 |

The above composition was mixed with a composition comprising hydrogen peroxide at a concentration of 2% by weight at a weight ratio of 1:1 which was resulted in a pH value of 6.9 and was applied onto a twice bleached hair. After processing of 30 min at 30°C the hair was rinsed with tap water and shampooed once with a commercial shampoo and dried with a hair drier. A shiny warm homogeneous colour was obtained. The L, a and b values measured at the root and tip parts as identified on the tress used were not significantly differing from each other showing clearly that hair is homogeneously coloured. In addition the hair feel soft and smooth upon touching and was easily combed through.

## Claims

1. Process for colouring hair wherein a composition based on oxidative dye precursors and coupling substances and comprising at least one branched fatty alcohol, at least one non-ionic emulsifier and at least one compound according to the formula 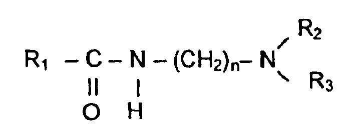 wherein R1 is an saturated or unsaturated alkyl chain having 11 to 23 C atoms, R₂ and R₃ are the same of different an alkyl chain having 1 to 4 C atoms and n is a number between 1 and 4 and/or its salts is mixed with an oxidizing composition comprising at least one oxidizing agent, preferably hydrogen peroxide and is applied onto hair and processed for 10 to 45 min at a temperature of 20 to 45°C and rinsed off from hair.

2. Process according to claim 1 **characterised in that** at least one branched fatty alcohol is chosen from octyldecanol, octyldodecanol, isocetyl and isostearyl alcohols.

3. Process according to claims 1 and 2 **characterised in that** at least one non-ionic emulsifier is a fatty alcohol ethoxylate.

4. Process according to any of the preceding claims **characterised in that** the compound according to the formula in claim 1 is chosen from stearamidopropyldimethylamine, palmitamidopropyldiethylamine, palmitamidopropyldimethylamine, behenamidopropyldimethylamine and /or their salts.

5. Process according to any of the preceding claims **characterised in that** the composition comprises at least one direct dye.

6. Process according to any of the preceding claims **characterised in that** the composition comprises additional fatty alcohol

7. Process according to any of the preceding claims **characterised in that** the composition comprises at least one cationic surfactant of the following formula 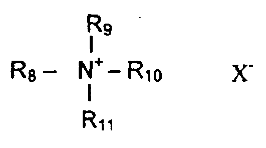 where R₈ is a saturated or unsaturated, branched or non-branched alkyl chain with 8-22 C atoms or
R₁₂ CO NH (CH₂)ₙ
where R₁₂ is saturated or unsaturated, branched or non-branched alkyl chain with 7-21 C atoms and n has typical value of 1 - 4 or
R₁₃ CO O (CH₂)ₙ
where R₁₃ is saturated or unsaturated, branched or non-branched alkyl chain with 7-21 C atoms and n has typical value of 1 - 4, and
R₉ is H or unsaturated or saturated, branched or non-branched alkyl chain with 1 - 22 C atoms or
R₁₂ CO NH (CH₂)ₙ
or
R₁₃ CO O (CH₂)ₙ
where R₁₂, R₁₃ and n are same as above,
R₁₀ and R₁₁ are H or lower alkyl chain with 1 to 4 Carbon atoms, and X is typically chloride, bromide, methosulfate.

8. Process according to any of the preceding claims **characterised in that** the composition comprises at least one organopolysiloxane wherein at least one silicium atom is linked to an alkylene group having a hetero-atom, in particular a nitrogen atom, with a poly-(N-acyl alkyleneimine) units of the formula 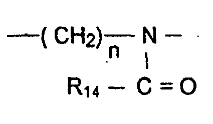 wherein n is a number from 1 to 5 and R₁₄ is hydrogen, a C₁-C₁₂-alkyl or cycloalkyl, aralkyl or aryl group.

## Patentansprüche

1. Verfahren zum Färben von Haaren wobei eine Zusammensetzung basierend auf oxydativen Farbentwicklern und Kupplerverbindungen, und mindestens einen verzweigten Fettalkohol, mindestens einen nicht ionischen Emulgator
und mindestens eine Verbindung entsprechend der Formel 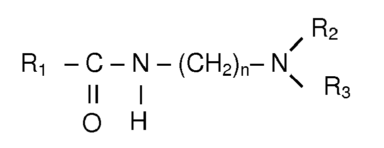 enthält,
worin R1 eine gesättigte oder ungesättigte Alkylkette mit 11 bis 23 C- Atomen ist, R₂ und R₃ sind gleiche oder unterschiedliche Alkylketten mit 1 bis 4 C-Atomen und n ist eine Zahl zwischen 1 bis 4 und/oder deren Salze, mit einer oxydierenden Zusammensetzung die mindestens eine oxydierende Verbindung, vorzugsweise Wasserstoffperoxyd, enthält, gemischt und auf das Haar aufgetragen wird und für 10 bis 45 Minuten bei einer Temperatur von 20 bis 45°C einwirkt und dann vom Haar abgespült wird.

2. Verfahren nach Anspruch 1, **dadurch gekennzeichnet, dass** mindestens ein verzweigter Fettalkohol aus Octyldecanol, Octyldodecanol, Isocetyl- und Isostearylalkoholen ausgewählt wurde.

3. Verfahren nach den Ansprüchen 1 und 2, **dadurch gekennzeichnet, dass** mindestens ein nicht ionischer Emulgator ein Fettalkoholethoxylat ist.

4. Verfahren nach einem der vorhergehenden Ansprüche, **dadurch gekennzeichnet, dass** die Verbindung entsprechend der Formel in Anspruch 1 aus Stearamidopropyldimethylamin, Palmitamidopropyldiethylamin, Palmitamidopropyldimethylamin, Behenamidopropyldimethylamin und /oder ihrer Salze ausgewählt ist.

5. Verfahren nach einem der vorhergehenden Ansprüche, wobei die Zusammensetzung mindestens einen direkt ziehenden Farbstoff enthält.

6. Verfahren nach einem der vorhergehenden Ansprüche, wobei die Zusammensetzung zusätzlich Fettalkohol enthält.

7. Verfahren nach einem der vorhergehenden Ansprüche, wobei die Zusammensetzung mindestens ein kationisches Tensid der folgenden Formel enthält 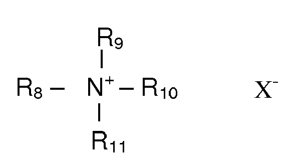 worin R₈ eine gesättigte oder ungesättigte, verzweigte oder nicht verzweigte Alkylkette mit 8 - 22 C-Atomen ist, oder
R₁₂ CO NH (CH₂)n
worin R₁₂ eine gesättigte oder ungesättigte, verzweigte oder nicht verzweigte Alkylkette mit 7 - 21 C- Atomen ist und n einen typischen Wert von 1 - 4 hat, oder
R₁₃ CO O (CH₂)n
worin R₁₃ eine gesättigte oder ungesättigte, verzweigte oder nicht verzweigte Alkylkette mit 7 - 21 C- Atomen ist und n einen typischen Wert von1 - 4 hat, und
R₉ ist H oder eine ungesättigte oder gesättigte, verzweigte oder nicht verzweigte Alkylkette mit 1 - 22 C- Atomen, oder
R₁₂ CO NH (CH₂)n
oder
R₁₃ CO O (CH₂)n
worin R₁₂, R₁₃ und n das gleiche wie oben sind,
R₁₀ und R₁₁ H oder eine niedrige Alkylkette mit 1 - 4 Kohlenstoffatomen sind und X typischer weise Chlorid, Bromid, Methosulfat ist.

8. Verfahren nach einem der vorhergehenden Ansprüche, wobei die Zusammensetzung mindestens ein Organopolysiloxane enthält, worin mindestens ein Siliziumatom an eine Alkylengruppe mit einem Heteroatom speziell einem Stickstoffatom mit einer Poly-(N-acyl alkyleneimine)- Einheit der Formel 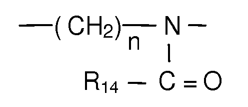 gebunden ist,
worin n eine Zahl von 1 bis 5 ist und R₁₄ Wasserstoff, eine C₁-C₁₂-Alkyl- oder Cycloalkyl-, Aralkyl oder Aryl- Gruppe ist.

## Revendications

1. Procédé de coloration des cheveux dans lequel une composition basée sur des précurseurs de colorants oxydatifs et des substances de couplage et comprend au moins un alcool gras ramifié, au moins un émulsifiant non ionique et au moins un composé selon la formule 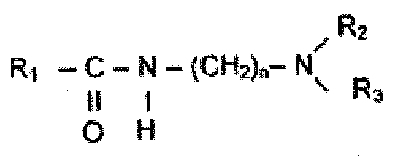 dans laquelle R₁ est une chaîne alkyle saturée ou insaturée ayant 11 à 23 atomes de C, R₂ et R₃ sont identiques ou différents une chaîne alkyle ayant 1 à 4 atomes de C et n est un nombre entre 1 et 4 et/ou ses sels est mélangée avec une composition oxydante comprenant au moins un agent oxydant, préférablement du peroxyde d'hydrogène, et appliquée sur les cheveux et ceux-ci sont traités pendant 10 à 45 minutes à une température de 20 à 45°C et rincés.

2. Procédé selon la revendication 1 **caractérisée en ce qu'**au moins un alcool gras ramifié est choisi parmi l'octyldécanol, l'octyldodécanol, les alcools isocétylique et isostéarylique.

3. Procédé selon les revendications 1 et 2 **caractérisée en ce qu'**au moins un émulsifiant non ionique est un éthoxylate d'alcool gras.

4. Procédé selon l'une quelconque des revendications précédentes **caractérisée en ce que** le composé selon la formule de la revendication 1 est choisi parmi la stéaramidopropyldiméthylamine, la palmitamidopropyldiéthylamine, la palmitamidopropyldiméthylamine, la béhènamidopropyldiméthylamine et/ou leurs sels.

5. Procédé selon l'une quelconque des revendications précédentes **caractérisée en ce qu'**elle comprend au moins un colorant direct.

6. Procédé selon l'une quelconque des revendications précédentes **caractérisée en ce qu'**elle comprend un alcool gras additionnel.

7. Procédé selon l'une quelconque des revendications précédentes **caractérisée en ce qu'**elle comprend au moins un surfactant tensioactif cationique de la formule suivante 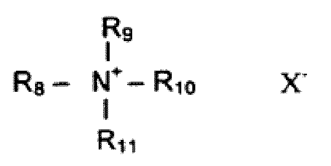 dans laquelle R₈ est une chaîne alkyle saturée ou insaturée, ramifiée ou non ramifiée avec 8 à 22 atomes de C ou
R₁₂CONH(CH₂)ₙ
dans laquelle R₁₂ est une chaîne alkyle saturée ou insaturée, ramifiée ou non ramifiée avec 7 à 21 atomes de C et n a une valeur type de 1 à 4 ou
R₁₃COO (CH₂)ₙ
dans laquelle R₁₃ est une chaîne alkyle saturée ou insaturée, ramifiée ou non ramifiée avec 7 à 21 atomes de C et n a une valeur type de 1 à 4 et
R₉ est un H ou une chaîne alkyle ramifiée ou non ramifiée, saturée ou insaturée avec 1 à 22 atomes de C ou
R₁₂CONH (CH₂) ₙ
ou
R₁₃COO(CH₂) ₙ
où R₁₂, R₁₃ et n sont les mêmes que ci-dessus,
R₁₀ et R₁₁ sont un H ou une chaîne alkyle inférieure avec 1 à 4 atomes de carbone, et X est typiquement un chlorure, un bromure, un méthosulfate.

8. Procédé selon l'une quelconque des revendications précédentes **caractérisée en ce qu'**elle comprend au moins un organopolysiloxane dans lequel au moins un atome de silicium est lié à un groupe alkylène ayant un hétéroatome, en particulier un atome d'azote, avec des unités poly-(N-acylalkylèneimine) de la formule 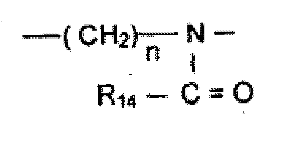 dans laquelle n est un nombre de 1 à 5 et R₁₄ est un hydrogène, un groupe alkyle ou cycloalkyle en C₁-C₁₂, un groupe aralkyle ou aryle.
